# EUROPÄISCHE PATENTSCHRIFT

(11) **EP 4 152 938 B1**
(45) Veröffentlichungstag und Bekanntmachung des Hinweises auf die Patenterteilung: **13.08.2025**
(21) Anmeldenummer: 21727136.0
(22) Anmeldetag: 19.05.2021
(51) Int. Cl.: A23C 11/10, A23J 1/14, A23L 11/00, A23L 11/65

(54) **VERFAHREN ZUR GEWINNUNG VON PROTEINEN AUS EINEM NATIVEN STOFFGEMENGE AUS SOJA ODER AUS SOJAMILCH**
METHOD FOR OBTAINING PROTEINS FROM A NATURAL MIXTURE OF SUBSTANCES FROM SOY OR FROM SOY MILK
PROCÉDÉ D'OBTENTION DE PROTÉINES À PARTIR D'UN MÉLANGE NATUREL DE SUBSTANCES DE SOJA OU DE LAIT DE SOJA

(30) Priorität: 20.05.2020 DE 102020113747
(43) Veröffentlichungstag der Anmeldung: 29.03.2023
(73) Patentinhaber: GEA Mechanical Equipment GmbH, 59302 Oelde (DE)
(72) Erfinder: ULLMANN, Detlef, 59302 Oelde (DE); SPEISER, Ines, 33415 Verl (DE); KROHN, Sabine, 59302 Oelde (DE); PECORONI, Stefan, 59302 Oelde (DE); HRUSCHKA, Steffen, 59302 Oelde (DE)
(74) Vertreter: Specht, Peter
(86) Internationale Anmeldenummer: PCT/EP2021/063341
(87) Internationale Veröffentlichungsnummer: WO 2021/234026

(56) Entgegenhaltungen:
- WO-A1-2006/129647
- CA-A1- 2 645 332
- CA-A1- 3 083 157
- US-A1- 2010 112 187

## Beschreibung

Die vorliegende Erfindung betrifft ein Verfahren zur Gewinnung von Proteinen aus Sojamilch. Die Sojamilch ist im Unterschied zum einzelhandelsüblichen Produkt keine umfangreich mit Wasser verdünnte Zusammensetzung, sondern ein dickflüssiges Produkt, welches unmittelbar mit einer Zentrifuge, insbesondere aus Sojabohnen, abgeschieden wird. Diese Sojamilch wird in der Fachliteratur oft auch als "soja base" (Sojabasis) oder "soy bean juice" (Sojabohnensaft) bezeichnet. Diese Sojamilch kann dabei auch beim Verfahren mit dem nativen Stoffgemenge aus Soja ein Zwischenprodukt sein.

Es ist bekannt, das gebildete Okara, das aus Soja-Pflanzenbestandteilen, z.B. aus Sojabohnen (geschält oder ungeschält) oder aus Soja-Flakes, gewonnen wurde, weiterzuverarbeiten. Aus der abgetrennten Sojamilch kann eine Proteinphase gewonnen werden.

Bislang bekannte Trennungsmethoden von Proteinen aus Sojamilch ermöglichen eine Gewinnung von etwa 80 Gew.% der in der Sojamilch enthaltenen Proteine . Dabei werden allerdings Öl und Ölbegleitstoffe wie Lipoxygenasen ins Protein überführt. Das vorgenannte Enzym reagiert mit dem vorhandenen Öl und bildet einen nachteiligen Geschmack in der Proteinphase. Neben Lipogenasen kann die Sojamilch u.a. auch Trypsin-Inhibitor als Inhaltsstoff aufweisen, welcher die Aufnahme von Proteinen im Körper stört. Ferner wird zum Schutz vor Ranzigkeit im Endprodukt ein minimaler Ölgehalt gefordert. WO 2006/129647 offenbart ein Verfahren zur Herstellung von Sojaprotein aus Sojamilch, wobei die Sojabohnen vorab entölt wurden.

Die WO 2007/113 176 A2 offenbart die Verarbeitung von Sojabohnen zur Herstellung von Sojamilch und nativen Proteinen. Eine Verringerung des Ölgehalts wurde bei diesem Verfahren nicht vorgenommen.

Die EP 1 905 312 A1 schlägt ebenfalls eine Separierung von Sojabohnen in Sojamilch und eine Proteinfraktion vor. Zur Entfernung von Öl aus dem Protein wurde eine Etherextraktion anstelle von Hexan eingesetzt.

Die EP 2 717 711 B1 geht in Abs. 0010 von einer wässrigen Proteindispersion aus, welche mit einer unvollständigen Verdrängungsextraktion im alkalischen Millieu unter Zugabe eines alkoholischen Lösungsmittels behandelt wird, um die Ölbegleitstoffe wie Lezithin in Lösung zu bringen. Damit wird die Abtrennung von gelösten oder dispergierten Proteinen von den Schalenbestandteilen möglich.

Die DE 10 2013 114 698 A1 offenbart schließlich ein Verfahren zur Proteingewinnung aus Leguminosen, wie z.B. Sojabohnen. Dabei werden ganze Bohnen zerkleinert und zu einem Brei verarbeitet. Dieser wird sodann schließlich nach einer pH-Verschiebung auf pH >9 unter Verwendung von Alkohol einer Trennung zum Entfernen der Schalen unterzogen. Schließlich erfolgt bei saurem pH-Wert eine Fällung und eine Abtrennung von mehreren Fraktionen darunter auch eine Protein- und eine Ölfraktion.

Es ist nunmehr die Aufgabe der vorliegenden Erfindung den Ölgehalt in der aus Sojabestandteilen gewonnenen Proteinphase derart zu senken, dass keine unerwünschten geschmacklichen Veränderungen auftreten.

Die vorliegende Erfindung löst diese Aufgabe durch das Bereitstellen eines Verfahrens mit den Merkmalen des Anspruchs 1 .

Das erfindungsgemäße Verfahren betrifft die Gewinnung von Proteinen aus Sojamilch, umfassend
i. ein Bereitstellen von Sojamilch
ii. Zugabe eines wäßrigen Alkohols mit einer Konzentration von weniger als 80 Vol.% zur Sojamilch unter Ausbildung einer organisch-wässrigen Suspension derart, dass durch die Zugabe wäßrigen Alkohols eine Verschiebung des Löslichkeitsgleichgewichtes stattfindet und eine Verdrängungsextraktion erfolgt, wobei das Volumen des wäßrigen Alkohols, so gewählt ist, dass der Gehalt des organischen Lösungsmittels der organisch-wässrigen Suspension nach Schritt ii. zumindest 15 Vol. % beträgt und dass der TS-Gehalt der Suspension mindestens 5 %, vorzugsweise zumindest 9 %, beträgt;
iii. Abtrennen einer Ölphase bzw. von Öl aus der Suspension
iv. Koagulieren der Proteine unter Ausbildung zumindest einer Proteinphase durch Einstellen der Suspension auf einen pH-Wert von weniger als pH=7 oder durch Zugabe eines proteinkoagulatbildenden Salzes;
   und
v. ein Abtrennen der Proteinphase mit einem Restölgehalt von unter 5 Gew.%, insbesondere mit einem Restölgehalt von unter 3 %, besonders bevorzugt mit einen Restölgehalt von unter 1 %, bezogen auf den Trockensubstanzgehalt der Proteinphase aus der Suspension.

Die Proteinphase weist geringe geschmackliche Veränderungen und einen geringen Anteil an Restöl auf.

Vorteilhafte Ausgestaltungen der Erfindung sind Gegenstand der Unteransprüche.

Das Bereitstellen von Sojamilch in Schritt i. kann durch Gewinnung von Sojamilch aus einem nativen Stoffgemenge aus Soja erfolgen, wobei das native Stoffgemenge zunächst zerkleinert und durch pH-Werteinstellung und Zugabe eines polaren Lösungsmittels, insbesondere von Wasser, zu einem fließfähigen alkalischen Brei verarbeitet wird, der zusätzlich zu Lipiden auch Proteine, Lecithin und Feststoffe enthält, wobei die Bearbeitung des Breis unter Ausbildung zweier voneinander getrennter Fraktionen in Form von Okara und von Sojamilch erfolgt.

Der Brei enthält alle wesentlichen Inhaltsstoffe der Sojabohne, auch Öl. Wenn die Sojabohne geschält ist, enthält der Brei weniger Ballaststoffe. Okara ist der "Feststoffkuchen" mit unlöslichen Bestandteilen und die Sojamilch weist viel Protein mit den gelösten und dispergierten Stoffen auf.

Zudem kann das Bereitstellen der Sojamilch ein Einengen, vorzugsweise Eindicken, von Sojamilch unter Erhöhung des Trockensubstanzgehalts umfassen, so dass die Bereitgestellte Sojamilch einen Trockensubstanzgehalt von bevorzugt zumindest 12 Gew.%, vorzugsweise zumindest 15 Gew.% aufweisen kann. Dies entspricht einer vergleichsweise relativ dickflüssigen Sojamilch.

Eine Einengung auf einen Trockensubstanzgehalt von mindestens 15% hat sich als besonders effizienter Weg erwiesen, bei den vorgeschriebenen Bedingungen von pH-Wert und Temperatur und Alkoholkonzentration die Ölanteile aus der Proteinstruktur herauszulösen. Proteinen umfassen bekanntermaßen eine Primär-, Sekundär-, Tertiär- und Quartärstruktur, welche Ölbestandteile typischerweise zurückhalten. Durch Öffnung der Struktur unter bestimmten Bedingungen ist eine besonders effiziente Abtrennung von Öl möglich.

Durch das Einengen erfolgt eine weiter-optimierte Vorbereitung der Proteinstruktur, sodann kann die Ölabtrennung durch die Verdrängungsextraktion mit EtOH noch erfolgreicher umgesetzt werden, Die Zugabe von Ethanol reduziert natürlich wieder den TS-Gehalt, vorzugsweise auf einen Trockensubstanzgehalt von zumindest 8 %.

Beim Einengen kann sich ggf. auch die innere Oberfläche der Öltröpfchen verändern und dadurch ein besseres Agglomerieren ermöglichen. Wenn die Öltröpfchen hinreichend groß sind können diese besonders gut zur kontinuierlichen Phase im Zentrifugalfeld agglomerieren und als solche abgetrennt werden.

Das Einengen kann besonders bevorzugt derart erfolgen, dass die Menge an entferntem Wasser in etwa der Menge an zudosiertem wäßrigen Alkohol entspricht. Somit ergibt sich trotz Zudosierung einer größeren Menge von verdünntem Alkohol zur Einstellung eines Alkoholgehalts von zumindest 15 Vol% ein TS-Wert von zumindest 8%.

Als Konzentration an zudosiertem Alkohol empfiehlt sich ein Alkohol mit weniger oder gleich 80 Vol.%. Höher konzentrierte Alkohole denaturieren die Proteine. Alkohole unterhalb von 50 Vol.% hingegen geben bei der Verdrängungsextraktion aufgrund des hohen Verdünnungsfaktors nur schlechte Ergebnisse.

Im Folgenden wird die weitergehende Verarbeitung der Sojamilch erläutert.

Die Zugabe des organischen Lösungsmittels erfolgt erfindungsgemäß als verdünnter Alkohol, vorzugsweise mit einem Alkohol-Anteil in Vol.% von zumindest 30%, vorzugsweise zwischen 50-80% erfolgen.

Bei 50 -80 Vol.% kann eine Rückgewinnung des Alkohols vorteilhaft unter Vakuum erfolgen.

Bevorzugt ist das organische wasserlösliche Lösungsmittel ein aliphatischer Alkohol, insbesondere mit einer Kettenlänge von weniger als sechs-Kohlenstoff-Atomen, und/oder Isopropanol.

Die pH-Einstellung in Schritt iv. kann vorzugsweise auf einen pH-Wert von mehr als pH=3,5, vorzugsweise zwischen pH= 3,8 bis 6,0, insbesondere zwischen pH=4,2 und 4,7, erfolgen.

Das Einengen kann durch Reduzierung des Wassergehalts der Sojamilch erfolgen, wobei das Gewicht um zumindest 20 Gew.%, vorzugsweise zumindest 40 Gew.%, auf einen TS-Gehalt von zumindest 12 Gew.%, besonders bevorzugt zumindest 15 Gew.% reduziert werden.

Der Gehalt des organischen wasserlöslichen Lösungsmittels in der Suspension nach Schritt iii. kann mehr als 15 Vol. %, vorzugsweise zwischen 25-45 Vol.%, betragen. Dadurch kann eine klare Ölphase abgetrennt werden. Bei geringeren Konzentrationen erfolgt ein Abtrennen einer ölhaltigen Phase in der Form einer Creme, was allerdings mit einem Produktverlust an Proteinen verbunden ist.

Sofern Sojamilch in Schritt i. aus einem fließfähigen okarahaltigen Brei hergestellt wird, so ist es von Vorteil, wenn der fließfähige alkalische Brei in Schritt i. bzw. die daraus bereitgestellte Sojamilch einen pH-Wert von mehr als pH=8, insbesondere zwischen pH=8,2 bis 9,8, aufweist. Es kann allerdings auch in einer weiteren Variante der Erfindung von einer sauren Sojamilch ohne vorherige pH-Werteinstellung mit einem pH-Wert um die 6,7 ausgegangen werden. Auch bei dieser Sojamilch empfiehlt sich eine Einstellung des pH-Werts für die Fällung nach erfolgter Ethanolzugabe, insbesondere in den bevorzugten Bereich von pH=3,8 bis 6,0.

Nach der Zugabe des wasserlöslichen organischen Lösungsmittels in Schritt iii., insbesondere unmittelbar nach der Zugabe des wasserlöslichen organischen Lösungsmittels, kann eine Abfolge aus zumindest einem Malaxieren, vorzugsweise mit einer Rührgeschwindigkeit von weniger als 100 U/min erfolgen. Die Abfolge kann besonders bevorzugt zudem zumindest einem Intensiv-Rühren, vorzugsweise mit einer Rührgeschwindigkeit von mehr als 500 U/min, umfassen. Die Reihenfolge innerhalb der Abfolge kann auch umgekehrt erfolgen, also zuerst Intensiv-Rühren und anschließend Malaxieren sein oder eine mehrfache Abfolge von Intensiv-Rühren, auf welche ein einmaliges Malaxieren folgt oder aber eine Abfolge von Malaxieren, auf welche ein einmaliges Intensiv-Rühren folgt. Durch diese Abfolge wird eine besonders vollständige Abscheidung von Öl erreicht, indem Öltröpfchen besser von den Proteinmolekülen abgetrennt werden und anschließend durch langsames Rühren zu größeren Öltröpfchen und schließlich zu einer Ölphase agglomerieren.

Unabhängig von den Wiederholungen der Abfolgen oder ob diese Abfolge überhaupt erfolgt sollte immer ein Malaxieren erfolgen und dieses sollte vorzugsweise die Abfolge beenden.

Das Malaxieren kann vorzugsweise bei einer Rührgeschwindigkeit von weniger als 50 U/min, vorzugsweise zwischen 10-30 U/min erfolgen. Das Malaxieren kann zudem für eine besonders optimale Agglomeration von Öl bei 20-70°C, besonders bevorzugt bei 40-70°C, erfolgen.

Bevorzugt kann das Malaxieren in einem Zeitintervall zwischen 5-30 min erfolgen. Längeres Malaxieren hat keinen weitergehenden vorteilhaften Effekt auf die Bildung der Ölphase.

Das Intensiv-Rühren hingegen kann bei Rührgeschwindigkeiten größer als 1000 U/min, insbesondere zwischen 1000-12000 U/min, erfolgen.

Die vorgenannte Abfolge kann mehrfach, insbesondere zumindest 3-mal, wiederholt werden, um eine zusätzliche Optimierung der Ölabscheidung aus der Suspension zu erreichen. Die vorgenannte Abfolge sollte jedoch idealerweise zumindest ein Malaxieren umfassen.

Weiterhin kann vor Schritt iv. und vorzugsweise nach der Abfolge aus zumindest einem Malaxieren und zumindest einem Intensiv-Rühren, ein Abtrennen, insbesondere ein zentrifugales Abtrennen, einer Ölphase aus der Suspension erfolgen. Aus Gründen der hohen Viskosität der Suspension bietet sich hierfür vorteilhaft ein Dekanter, insbesondere in der Ausgestaltung einer Vollmantelschnecken-Zentrifuge, oder ein Separator an.

Das zentrifugale Abtrennen kann besonders optimal bei einer Temperatur von mehr als 20°C, vorzugsweise zwischen 35-80°C, besonders bevorzugt zwischen 35-45°C, erfolgen. Speziell in den vorgenannten Bereichen hat sich ein Optimum aus Verarbeitungszeit und technischem Aufwand (Ex-Betrieb) ergeben.

Vor Schritt iv. und vorzugsweise nach dem zentrifugalen Abtrennen der Ölphase kann eine Entalkoholisierung, vorzugsweise durch Verdampfen des Alkohols, erfolgen. Dabei kann eine Rückgewinnung des zuvor zugeführten organischen Lösungsmittels in Form von Alkohol erfolgen.

Nach der pH-Einstellung in Schritt iv. kann die Suspension in einen Behälter zur Fällung des Proteins überführt werden.

Das Fällen kann bei einer Temperatur von mehr als 20°C, vorzugsweise zwischen 50-80°C, besonders bevorzugt zwischen 60-75°C und ebenfalls vorzugsweise in einem Zeitraum zwischen 5-20 min erfolgen.

Das Abtrennen in Schritt v. kann ein zentrifugales Abtrennen sein und vorzugsweise durch einen Dekanter, insbesondere durch eine Vollmantel-Schneckenzentrifuge, durchgeführt werden.

Das zentrifugale Abtrennen kann in einer Zentrifuge oder einem Dekanter mit einem Abtrennbereich, dem sogenannten cut-off value, zwischen 1,0 bis 100 µm, erfolgen.

Im Anschluss an die Proteintrennung kann eine Trocknung erfolgen.

Die in Schritt v. abgetrennte Proteinphase kann weniger als 0,5 % Gew. Lecithin aufweisen.

Die Schritte i. bis iii. und vorzugsweise auch das Bereitstellen des alkalischen Breis, können in einem Temperaturbereich zwischen 35°C bis maximal 85°C erfolgen.

Das polare Lösungsmittel zur Bildung des fließfähigen Breis vor Schritt i. kann vorzugsweise Wasser sein, wobei das Volumen an -wässrigen polaren Lösemittel so gewählt wird, dass sich ein Verdünnungsfaktor von mehr als 0,2, vorzugsweise zumindest 2,0 einstellt, bezogen auf das eingeengten Brei.. Die Rohsuspension hat üblicherweise einen Trockensubstanzgehalt von ca. 8-9% TS, dann wird sie eingeengt und wieder mit polarem Lösungsmittel verdünnt. Dann sollte sie wieder TS-Gehalt von nicht unter 5%, vorzugsweise zwischen 7% bis 13%, besonders bevorzugt zumindest 8 %, insbesondere 9% (+/- 1), haben. Ein höherer Trockensubstanzgehalt ist jedoch nicht nachteilig.

Für einen Trockensubstanzgehalt von mehr als 5% empfiehlt sich besonders für bei Temperaturen um die 70°C, während bei einem Trockensubstanzgehalt von zumindest 8 % auch bei Temperaturen von weniger als 70 °C gearbeitet werden kann, was prozesstechnisch vorteilhaft ist.

Das Zerkleinern des nativen Stoffgemenges kann produktschonend durch eine Nassvermahlung erfolgen.

Das Abtrennen in Schritt v. kann innerhalb von 60 min, insbesondere innerhalb von 30 min, nach dem Durchführen des Schritts iv. begonnen werden, um Nebenreaktionen zu vermeiden.

Die Gewinnung der Proteinphase erfolgt insbesondere ohne Zugabe von Hexan, so dass keine noch gesundheitliche Bedenken bei der Verwendung des Endproduktes als Futtermittel oder Nahrungsmittels auftreten.

Die Gewinnung und Verarbeitung von Ölen und Fetten nach den Richtlinien des ökologischen Landbaus erlaubt insbesondere mechanische Produktionsschritte. Der Einsatz von chemischen Hilfsmitteln ist bis auf wenige Ausnahmen verboten. Dazu zählen auch Extraktionslösemittel wie Hexan und Ether.

Das Einstellen des pH-Werts in Schritt iv. durch Zugabe einer organischen oder anorganischen Säure erfolgen, beispielsweise durch eine Fruchtsäure.

Eine bevorzugte Ausführungsvariante des Verfahrens weist die folgende Schrittabfolge auf:
i. Bereitstellen von Sojamilch, vorzugsweise mit einem pH-Wert von mehr als pH=7,2
ii Einengen von Sojamilch unter Erhöhung des Trockensubstanzgehalts;
iii. Zugabe eines kurzkettigen Alkohols mit 1-5 Kohlenstoffatomen unter Ausbildung einer wässrig-alkoholischen Suspension, insbesondere unter Zugabe von Ethanol und/oder Isopropanol, wobei der Alkoholgehalt der alkoholisch-wässrigen Suspension nach Schritt iii. zumindest 15 Vol. %, vorzugsweise zumindest 20 Vol.%, beträgt,
iii.i Abfolge aus zumindest einem Malaxieren mit einer Rührgeschwindigkeit von weniger als 100 U/min
iii.ii Abtrennen, insbesondere erstes zentrifugales Abtrennen, von Öl aus der Suspension unter Ausbildung einer im Wesentlichen ölfreien Suspension mit einem Ölgehalt von weniger als 5 Gew.%, vorzugsweise weniger als 3 Gew.% in der Trockenmasse;
iii.iii Optionales Verringern des Alkoholgehalts, vorzugsweise durch Destillation, besonders bevorzugt unter Rückgewinnung von Alkohol;
iv. Einstellen der Suspension auf einen pH-Wert von weniger als pH=7, vorzugsweise auf einen pH-Wert zwischen pH=3,5 bis pH=6; unter Ausbildung zumindest einer Proteinphase und zumindest einer Flüssigphase;
iv.i Optionales Fällen der Proteinphase als Feststoffphase;
v. Abtrennen, insbesondere zweites zentrifugales Abtrennen, einer Proteinphase mit einem Restölgehalt von unter 5 Gew.% aus der Suspension, vorzugsweise zwischen 1-3 Gew.%, bezogen auf die Trockensubstanz der Proteinphase, sowie
vi. Trocknen der Proteinphase zu einem Protein

Weiterhin beschrieben ist ein Verfahren zur Gewinnung von Proteinen aus einem nativen Stoffgemenge aus Soja, wobei das native Stoffgemenge zunächst zerkleinert und durch pH-Werteinstellung in den basischen Bereich und Zugabe eines polaren Lösungsmittels zu einem fließfähigen alkalischen Brei verarbeitet wird, der zusätzlich zu Lipiden auch Proteine, Lecithin und Feststoffe enthält, wobei das Verfahren die folgenden weiteren Schritte aufweist:
- A: Bearbeitung des Breis unter Ausbildung zweier voneinander getrennter Fraktionen in Form von Okara und von Sojamilch und
- B: Verarbeitung der Sojamilch zu einer Proteinphase nach den vorgenannten erfindungsgemäßen Verfahren.

Nachfolgend werden weitere vorteilhafte Ausgestaltungen des vorgenannten Verfahrens beschrieben. Die pH-Werteinstellung zur Erzeugung des fließfähigen alkalischen Breis kann vorzugsweise durch Zugabe einer Lauge, wie NaOH oder einer NaHCO₃-Lösung erfolgen.

Dieser Brei enthält zusätzlich zu Lipiden auch Proteine, Lecithin und Feststoffe.

Bei der Trennung in Sojamilch und Okara wird ein Großteil der Proteine aus dem Brei mit der Sojamilch gelöst und/oder dispergiert abgetrennt. Bei einem zusätzlichen Waschvorgang der Okara kann die Proteinausbeute in der Sojamilch auf deutlich über 70% gesteigert werden.

Ein Teil der Proteine verbleibt dabei im Okara und kann gesondert von der weiteren Verarbeitung der Sojamilch gewonnen werden.

Die Schritte i) bis iii), und vorzugsweise auch das Bereitstellen des alkalischen Breis, können bevorzugt in einem Temperaturbereich bis maximal 85°C, erfolgen. Insbesondere kann zumindest das Bereitstellen des alkalischen Breis bei weniger als 15°C erfolgen, um eine geschmackliche Veränderung des finalen Proteinproduktes oder weiterer Nebenprodukte, wie z.B. des Okara, nicht negativ zu beeinflussen.

Üblicherweise wird Sojamilch bereits bei der Bereitstellung gekocht oder erhitzt, um die Trypsin-Inhibitoren zu neutralisieren, da diese sonst bei Verzehr des Proteins die Verdauung stören würden. Dies kann optional auch beim Bereitstellen in Schritt i) erfolgen.

Das polare Lösungsmittel zur Bildung des fließfähigen Breis ist vorzugsweise Wasser, wobei das Volumen an Wasser zu Bohne so gewählt wird, dass sich ein Verdünnungsfaktor von mehr als 2,0, vorzugsweise zumindest 3,0, insbesondere zwischen 3,5 bis 7,5 einstellt. Bevorzugt kann das polare Lösungsmittel zur Bildung des Breis im Prozess, z.B. durch einen Verdampfer, zurückgewonnen werden. So ist es z.B. möglich nach der Abtrennung der Proteinphase in Schritt v. sowohl das polare Lösungsmittel zur Bildung des fließfähigen Breis, als auch den eingesetzten Alkohol, durch fraktionierte Destillation zurückzugewinnen und den verschiedenen Stufen des Prozesses zuzuführen. Dies reduziert u.a. auch die Entsorgungskosten.

Das Zerkleinern des nativen Stoffgemenges kann vorteilhaft und produktschonend durch eine Nassvermahlung erfolgen.

Das vorliegende Verfahren hat den besonderen Vorteil, dass zur Gewinnung der Proteinphase auf die Zugabe von Hexan und Ether vollumfänglich verzichtet werden kann. Dadurch müssen keine kosten- und arbeitsintensive Zusatzschritte durchgeführt werden, um diese Substanz wieder aus dem Produkt zu entfernen. Hexan ist gesundheitsschädlich und folglich in Produkten in der Futter- und Nahrungsmittelindustrie unerwünscht.

Weiterhin beschrieben ist ein Verfahren zur Gewinnung von Proteinen aus Sojamilch mit einem Restölgehalt von mehr als 3% in Trockensubstanz. Sojamilch enthält typischerweise immer einen Restölgehalt weit jenseits der 3% in TS meist um die 20% Öl/TS (Trockensubstanz). Die Sojamilch kann idealerweise nach dem vorgenannten Verfahren gewonnen werden, da diese Sojamilch besonders trockensubstanz- und proteinreich ist. Es kann allerdings auch eine Sojamilch mit dem erfindungsgemäßen Verfahren verarbeitet werden, welche nicht aus einem fließfähigen alkalisch-wässrigen Brei gewonnen wurde und gemäß dem Schritt i. des vorhergehend-beschriebenen Verfahrens abgetrennt wurde.

Es versteht sich, dass alle Varianten, insbesondere die als vorteilhaft beschriebenen Varianten der vorgenannten Schritte, welche beim beschriebenen Verfahren zur Gewinnung von Proteinen aus einem nativen Stoffgemenge aus Soja angewandt werden können, auch beim vorgenannten Verfahren zur Gewinnung von Proteinen aus Sojamilch ebenfalls vorteilhaft angewandt werden können.

Ebenfalls beschrieben ist ein Sojaproteinpulver mit einem Ölgehalt von max. 5% Öl in der Trockenmasse und einem Proteingehalt von mind. 70% Protein in der Trockenmasse, wobei das Sojaproteinpulver absolut hexanfrei ist. Da im vorgenannten Verfahren kein Hexan eingesetzt wird, ist auch im Produkt kein Hexan (auch nicht im ppm-Bereich) vorhanden. Ein vorgenanntes Produkt mit diesem Ölgehalt ist bislang nicht bekannt.

Weitere Vorteile, Merkmale und Einzelheiten der Erfindung ergeben sich aus der nachfolgenden Beschreibung, in der mehrere Ausführungsbeispiele der Erfindung anhand der beiliegenden Zeichnung näher erläutert werden.

Es zeigen:
Fig. 1 Ablaufdiagramm verschiedener Vorbereitungsschritte zur Bereitstellung von Sojamilch während der Herstellung von Protein aus Soja; und
Fig. 2 Ablaufdiagramm zur Herstellung eines Proteinpulvers aus Sojamilch als eine Variante des erfindungsgemäßen Verfahrens.

Proteine weisen zumeist eine Primär-, Sekundär-, Tertiär- und ggf. auch einer Quartärstruktur auf. Der Strukturaufbau richtet sich dabei nach den Inhaltsstoffen, wie dem Ölgehalt, der Art des Öls (Polarität), der Art und der Konzentration der weiteren Inhaltsstoffe in der Suspension, dem Verdünnungsfaktor, der Polarität des Lösungsmittels, dem pH-Wert, der Temperatur und vielen weiteren Faktoren. Der Strukturaufbau bestimmt, inwieweit einzelne Inhaltsstoffe wie Öl und andere unpolare sowie wenig polare Stoffe freigesetzt werden können. Liegt das Protein in einer kompakteren Struktur vor, so ist es schwieriger ein Öl aus der Proteinstruktur zu lösen. Daher können die Bedingungen der Proteinherstellung je nach der Pflanzensorte stark variieren.

Im nachfolgenden Verfahren wird eine optimierte Gewinnung von Proteinen aus Soja, insbesondere Sojabohnen, beschrieben. Das Verfahren kann alternativ auch erst bei einer Sojamilch als Zwischenprodukt ansetzen.

In einem optionalen ersten Schritt erfolgt ein Bereitstellen eines nativen Stoffgemenges aus Soja 1. Als natives Stoffgemenge aus Soja werden in der vorliegenden Erfindung insbesondere Sojabohnen im geschälten oder ungeschälten Zustand verstanden, aber auch sogenannte Soja-Flakes. Von einem Stoffgemenge spricht man bei Vorliegen von granularen Komponenten z.B. als Haufwerk oder als Schüttgut.

Ein solches Stoffgemenge aus Soja 1 kann beispielsweise aus 38 Gew.% Protein, 18 Gew.% Fett, 15 Gew.% unlösliche Kohlenwasserstoffe, 15 Gew.% lösliche Kohlenwasserstoffe und 14 Gew.% anderer Inhaltsstoffe bestehen

Das Stoffgemenge aus Soja kann zunächst in einem Lagersilo 2 aufbewahrt werden und sodann über eine Reinigungsanlage 3 geführt werden, welche das Soja, also die Sojabohnen oder -flakes, von Schmutz befreit und Steine und Besatz abtrennt. Dies kann z.B. durch Waschen und Sieben des Stoffgemenges erfolgen.

In einem optionalen zweiten Schritt kann ein Aufweichen 4 des nativen Stoffgemisches erfolgen. Hierfür kann Wasser 5, vorzugsweise Wasser einer Temperatur von weniger als 20 °C, vorzugsweise weniger als 15 °C, zu dem Stoffgemenge zugegen werden. Dies vermindert die Enzymaktivität, insbesondere die Lipoxygenase-Aktivität.

Das Aufweichen 4 erfolgt vorzugsweise innerhalb eines Zeitraumes von zumindest 3h, vorzugsweise von 4 bis 10 Stunden. Zum Aufweichen des Stoffgemisches empfiehlt sich bevorzugt die Verwendung von geschälten Sojabohnen oder Sojaflakes. Diese benötigen etwa nur 3,5 bis 4,5 h zum Aufweichen. Ungeschälte Sojabohnen benötigen länger zum Aufweichen. Das Aufweichen der Sojabohnen ermöglicht bessere Bedingungen bei der anschließenden Vermahlung. Das Vermahlen von aufgeweichten Pflanzenbestandteilen wird allgemein als Nassvermahlung bezeichnet.

Als eingesetzte Wassermenge 5 empfiehlt sich bezogen auf die Menge an Soja ein Gewichtsverhältnis von zumindest 2:1 (Wasser zu Soja) oder mehr, z.B. 3:1.

In einem dritten Schritt erfolgt ein Zerkleinern 6 der Komponenten des Sojas des Stoffgemenges 1. Dieses kann vorzugsweise als Nassvermahlung in Form einer Kaltvermahlung, Warmvermahlung oder Heißvermahlung erfolgen.

Die Kaltvermahlung erfolgt vorzugsweise bei Temperaturen von weniger als 15°C. Dies vermindert die Enzymaktivität, führt allerdings zur Verringerung der Ausbeute.

Die Warmvermahlung wird vorzugsweise bei Temperaturen zwischen 30-50°C, vorzugsweise bei etwa 40°C, durchgeführt. Dadurch erfolgt aufgrund der erhöhten Enzymaktivität eine Veränderung des Geschmacks als bohnenartiger Geschmack der anschließend hergestellten Sojamilch. Dieser kann bei einigen Verbraucherkreisen erwünscht sein. Zugleich ermöglicht die höhere Temperatur eine Ausbeuteverbesserung.

Die Heißvermahlung erfolgt bei Temperaturen von über 80°C. Dadurch werden Enzyme deaktiviert. Bei dieser Variante wird heißes Wasser, vorzugsweise bei mehr als 95°C, zu den Soja-Komponenten zugegeben und vorzugsweise für mehrere Minuten, z.B. zumindest 4 Minuten, gehalten. Auch bei dieser Variante verringert sich die Ausbeute im Vergleich zur Warmvermahlung.

Die Zerkleinerung 6 durch Vermahlung kann optional und bevorzugt zumindest zweistufig erfolgen. Die erste Stufe kann eine Vorvermahlung in einer Scheibenmühle erfolgen.

Sofern eine vorbestimmte mittlere Teilchengröße erreicht ist, kann eine weitere Feinvermahlung in einer Colloidmühle erfolgen. Dabei werden Zellen geöffnet und dadurch die Ausbeute erhöht.

Vor, während oder nach dem Zerkleinern kann in einem vierten Schritt eine Erhöhung des pH-Werts 7 des Gemenges oder des zerkleinerten Gemenges erfolgen. Dabei wird der pH-Wert aus Gründen einer Ausbeuteoptimierung erhöht. Der höhere pH-Wert ermöglicht u.a. eine verbesserte Löslichkeit der Proteine in Wasser. Zur Erhöhung des pH-Werts bietet sich bevorzugt die Zugabe einer Lauge, z.B. NaOH-Lösung, oder einer Pufferlösung 8 an, beispielsweise einer Natrium hydrogencarbonat-Lösung.

Das Ergebnis der vorgenannten Schritte ist ein fließfähiger alkalischer Brei 9, welcher in einem fünften Schritt zumindest in Okara 11 und Sojamilch 12 fraktioniert wird. Weiterhin vorteilhaft für die Ausbeute von ölfreiem Protein aus Sojamilch ist der Anteil der Wasserzugabe 5 bei der Herstellung des fließfähigen Breis 9. So haben Versuchsergebnisse gezeigt, dass bei einem Wechsel von einem Verdünnungsfaktor von 2 auf einen Verdünnungsfaktor von 3 bei der Wasserzugabe vor der Fraktionierung des Breis in Okara und Sojamilch, bis zu 10 % mehr Proteine als ölfreies Produkt aus der Sojamilch gewonnen werden können. Die 10 % mehr Proteine beziehen sich auf das Gesamtgewicht an Proteinen im Stoffgemenge als Ausgangsstoff. Eine weitere Erhöhung der Wassermenge bringt weitere Ausbeuteerhöhungen, jedoch wird ab einer Wassermenge größer 7 zu 1 der Prozess verfahrenstechnisch ungünstig.

Dabei wird zur Trennung 10 in die beiden vorgenannten Fraktionen zumindest ein erster Dekanter, vorzugsweise eine Vollmantelschneckenzentrifuge, eingesetzt. Der erste Dekanter hat vorzugsweise eine horizontale Lage oder eine zur horizontalen Lage bis zu 25° geneigte Drehachse. Die Zufuhr des Breis erfolgt axial und der Austrag der Feststoffe, also des Okara, ebenso wie der Austrag der Sojamilch erfolgt radial. Dabei ist kann der Austrag der Feststoffe in einem Bereich eines ersten Endes des Dekanters erfolgen und der Austrag der Sojamilch in einem Bereich eines zweiten Endes des Dekanters.

Neben der weiteren erfindungsgemäßen Behandlung von Sojamilch 12 kann auch Okara 11 in einem ein- oder mehrstufigen Prozess weiterverarbeitet werden. Im einstufigen Prozess wird das Okara 11, wie vorbeschrieben, im Dekanter von der Sojamilch getrennt und aus dem Dekanter abgeführt. Hier kann eine Schneckenpumpe zum Abtransport genutzt werden. Dabei kann eine Proteinausbeute für das Verfahren von über 70 % erreicht werden. Es ist alternativ auch möglich das abgetrennte Okara 11 nochmals in Wasser aufzuschlämmen und mit einem zweiten Dekanter nachzubehandeln. Das abgetrennte Soja-Wasser kann zum Aufweichen in den Prozess zurückgeführt werden. Die Proteinausbeute kann bei dieser Variante des Verfahrens bis zu circa 80% betragen.

Der Brei 9, welcher dem Zulauf des ersten Dekanters zugeführt wird, kann einen Anteil an unlöslichen Bestandteilen von 25-30 Vol.% aufweisen. Die Temperatur des zugeführten Breis kann bevorzugt mehr als 75°C betragen.

Der Trockensubstanzgehalt der Sojamilch 12 am Auslass des ersten Dekanters beträgt vorzugsweise >5 % m/m, vorzugsweise zumindest 8%, ideal bei 8 bis 10,5% m/m.

Optional kann die Sojamilch in einem nicht näher dargestellten optionalen sechsten Schritt durch Ultrahocherhitzen und/oder Deodorisierung behandelt werden. Dabei kann zur Deaktivierung der Enzymaktivität heißer Dampf in die Sojamilch eingeleitet werden. Das Produkt auf mehr als 100°C, vorzugsweise zwischen 120-140°C, erwärmt werden. Dadurch kann eine Deaktivierung des Trypsin-Inhibitors erreicht werden. Bevorzugt ist dabei wie beschrieben eine Direkterhitzung durch Dampfeinleitung. Die Temperatur wird weniger als 60 Sekunden, vorzugsweise weniger als 10 Sekunden gehalten. Danach kann die Soja-Milch wieder gekühlt werden. Dies kann vorzugsweise durch ein Vakuumkühlsystem zur Direktkühlung ohne zusätzliche Kältemittel in Form einer Entspannungskühlung erfolgen.

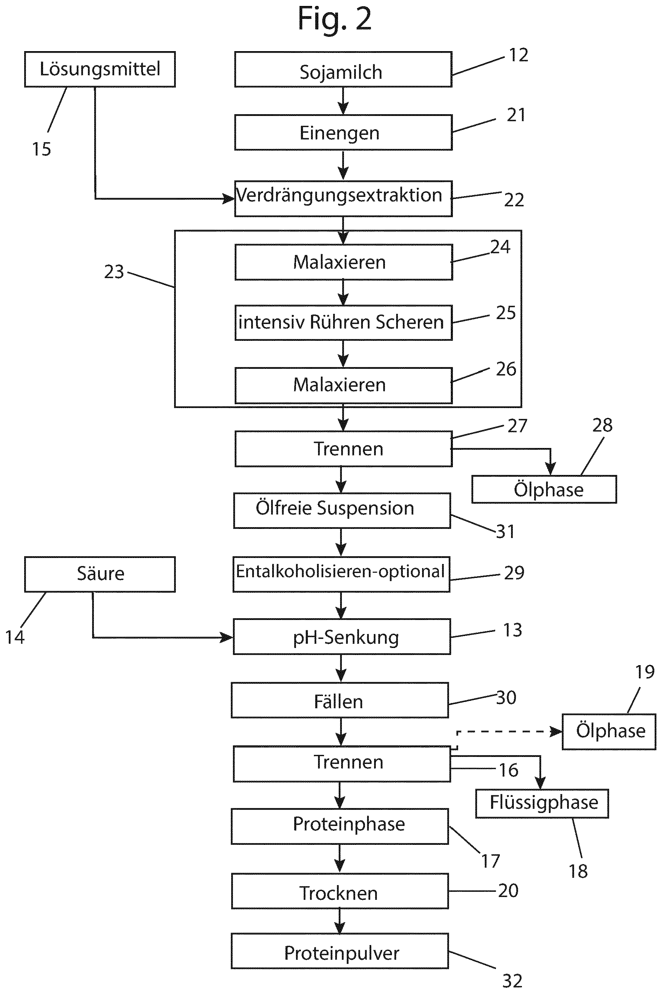

Eine erfindungsgemäße Ausführungsvariante zur Verarbeitung von Sojamilch wird in Fig. 2 dargestellt.

In einem siebenten Schritt (Schritt ii) erfolgt sodann ein Einengen 21 der Menge an Sojamilch z.B. durch Verdampfen eines größeren Anteils an Wasser aus der Sojamilch 12, so dass eine Reduzierung des Gewichts der Sojamilch um zumindest 10 Gew.%, vorzugsweise zumindest 40 Gew.%, erfolgt. Es gibt allerdings auch Sojamilch die schon mit 12 oder 12,5% TS als Rohstoff vorliegt. Diese muss nicht mehr so viel eingeengt werden. Insgesamt empfiehlt sich ein Produkt vor der Ethanolextraktion mit einem TS-Gehalt von zumindest 12%, vorzugsweise 15 %.

Dieser Schritt bevorzugt für die nachfolgende Phasenumkehr. Es hat sich überraschend gezeigt, dass eine Einengung der Menge an Sojamilch, insbesondere auf einen TS-Gehalt von 15 Gew.%, besonders vorteilhaft ist, um das Verfahren nur unter verfahrenstechnischen Gesichtspunkten zu optimieren und um das Öl in optimaler Weise freizusetzen.

Im Anschluss an das Einengen 21 im siebenten Schritt erfolgt in einem achten Schritt die Zugabe eines organischen Lösungsmittels 15 im Rahmen einer Verdrängungsextraktion 22. Als organisches Lösungsmittel 15 empfiehlt sich besonders bevorzugt ein Alkohol mit 1-5 Kohlenstoffatomen, besonders bevorzugt jedoch Ethanol und/oder Isopropanol. Es hat sich gezeigt, dass bei Zugabe eines verdünnten alkoholischen Lösungsmittels eine bessere Abtrennung von Proteinen mit geringem Ölgehalt möglich ist, als dies z.B. bei konzentriertem Alkohol der Fall ist. Die Zugabe des Lösungsmittels in verdünnter Form kann daher bevorzugt mit einem Lösungsmittel-Anteil in Vol.% von mehr als 30%, vorzugsweise zwischen 50-96%, besonders bevorzugt zwischen 55 bis 80%, erfolgen. Es hat sich beispielsweise für die Verwendung von Ethanol eine ideale Alkohol-Verdünnung von 60 Vol.% (+/- 5%) ergeben. Während der Zugabe des Lösemittels kann eine Vermischung der Suspension erfolgen, um eine schnelle Verteilung und homogene Verteilung in der Suspension zu erreichen.

Das Volumen und die Konzentration des Lösungsmittels, insbesondere des Alkohols, sind dabei so zu bemessen, dass der Gehalt des organischen wasserlöslichen Lösungsmittels 15 in der Suspension nach Schritt iii. mehr als 15 Vol. %, vorzugsweise zwischen 25-45 Vol.%, beträgt.

Es hat sich beispielsweise für die Verwendung von Ethanol eine ideale AlkoholKonzentration in der Sojamilch von 25 Vol.% (+/- 5%) ergeben.

Zu Vergleichszwecken wurde mit der gleichen Sojamilch 12 eine rein-wässrige Behandlung im sauren pH-Bereich (pH=5) ohne Ethanolzugabe durchgeführt. Dabei ergab sich nach dem Abtrennen der Feststoffphase ein Proteinprodukt mit einem Ölgehalt von 5,1 Gew.%.

Nach der Zugabe des organischen wasserlöslichen Lösungsmittels im achten Schritt erfolgt in einem neunten Schritt eine Abfolge 22 aus Malaxieren 24 und Intensiv-Rühren 25. Das Intensiv-Rühren 25 kann insbesondere ein Intensivmischen umfassen. Die Reihenfolge kann dabei beliebig gewählt werden. Insbesondere kann auch zuerst ein Intensiv-Rühren und anschließend ein Malaxieren erfolgen. Es ist vorteilhaft, unmittelbar vor dem Trennen 27 zumindest einen Malaxierschritt 26 auszuführen. Allerdings kann vor diesem Malaxierschritt 26, wie zuvor beschrieben auch eine Abfolge aus einem weiteren Malaxieren 24 und Intensiv-Rühren 25 in beliebiger Reihenfolge in mehrfacher Wiederholung erfolgen.

Ein Intensiv-Rühren 25 nach der Definition der vorliegenden Erfindung erfolgt bei einer Geschwindigkeit von mehr als 500 Umdrehungen pro Minute, besonders bevorzugt 1000-500 U/min.

Demgegenüber erfolgt ein Malaxieren 24, 26 nach der Definition der vorliegenden Erfindung bei einer Rührgeschwindigkeit von weniger als 100 U/min, vorzugsweise weniger als 50 U/min, besonders bevorzugt zwischen 10-30 U/min.

Beim Intensiv-Rühren treten aufgrund der erhöhten Rührgeschwindigkeit Scherkräfte auf, welche eine verbesserte Abtrennung des Öls von Proteinstrukturen ermöglichen. Dabei werden Mikroöltröpfchen quasi aus dem proteinhaltigen Material herausgepresst.

Demgegenüber ermöglicht das Malaxieren aufgrund der geringen Rührgeschwindigkeit eine Agglomeration der Mikroöltröpfchen in der Suspension zu einem größeren Öltropfen bis hin zu einer Ölschicht.

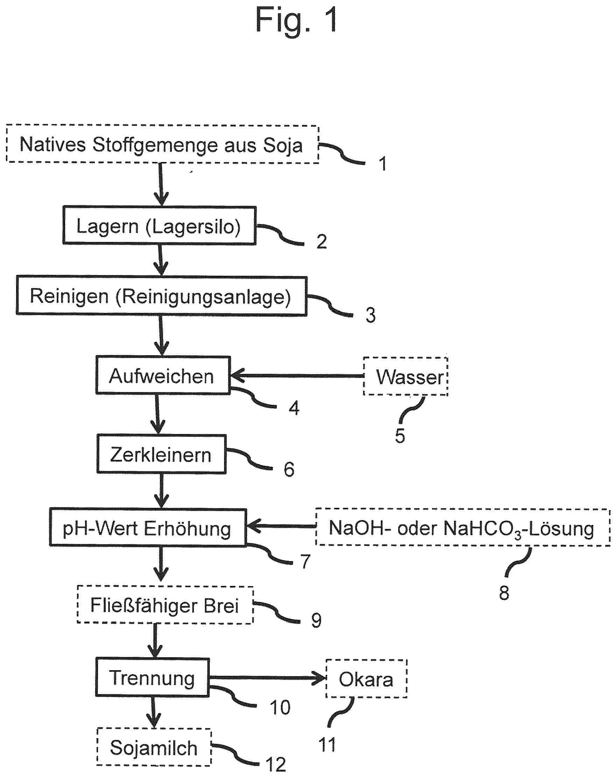

In Fig. 1 ist eine Abfolge aus Malaxieren 24, Intensiv-Rühren 25 gefolgt von einem Malaxieren 26 einmalig dargestellt. Diese Abfolge der Schritte 24 und 25 wird in Fig. 2 als Schritt 23 bezeichnet und kann auch 0 bis n mal ausgeführt werden. Dabei stellt n die Anzahl der Ausführungen beliebiger Reihenfolge dar.

Die Temperatur während des Malaxierens 24, 26 beträgt vorzugsweise 40-70°C und das Zeitintervall des Malaxierens 24, 26 beträgt ebenfalls vorzugsweise zwischen 5-30 min.

In einem zehnten Schritt erfolgt ein Abtrennen 27 einer Ölphase 28 unter Ausbildung einer im Wesentlichen ölfreien Suspension 31. Enthält die Suspension nach dem achten Schritt mehr als 20 Vol.% des Lösungsmittels, insbesondere des Alkohols, so kann in diesem Schritt eine vergleichsweise klare Ölphase 28 abgetrennt werden. Bei Konzentrationen an Lösungsmittel zwischen 15-20 Vol.% weist die abgetrennte Ölphase 28 die Konsistenz einer Creme auf.

Das Abtrennen kann vorzugsweise bei einer Temperatur von mehr als 40°C, vorzugsweise zwischen 50-80°C, besonders bevorzugt zwischen 60-75°C, beispielsweise bei 70°C, erfolgen. Dadurch wird das Abscheiden von Öl zusätzlich erleichtert.

Optional kann ein Entalkoholisieren 29 in einem elften Schritt erfolgen. Dieses Entalkoholisieren kann durch Verdampfen oder Destillieren von Alkohol erfolgen. Insbesondere ist es möglich den im achten Schritt eingesetzten Alkohol zumindest teilweise zurückzugewinnen. Das Entalkoholisieren ist allerdings nicht zwingend notwendig. Die Proteinausbeute ist ohne diesen Schritt vergleichbar.

Sodann erfolgt eine endgültige pH-Werteinstellung der basischen Suspension in einem zwölften Schritt 13, sofern diese nicht bereits vor Schritt 10 erfolgt ist. Die Einstellung des pH-Werts soll für eine Säurefällung eine Verschiebung in den sauren Bereich, also unter pH=7, ermöglichen.

Zum Einstellen des pH-Wertes wird vorzugsweise eine lebensmittelverträgliche Säure 14 eingesetzt. Dabei handelt es sich bevorzugt um eine Fruchtsäure und besonders bevorzugt um Zitronensäure. Allerdings ist auch die Verwendung einer anorganischen Säure, wie z.B. HCl, möglich. Der pH-Wert sollte vorzugsweise auf einen pH-Wert von mehr als pH=3,5, vorzugsweise zwischen pH=3,8 bis 6,0, insbesondere zwischen pH=4,2 und 4,7, erfolgen, da der Aufbau der Proteinstruktur in diesem Bereich eine optimale Ölabtrennung ermöglicht und die Proteine eine Struktur aufweisen, welche eine weitergehende Verarbeitung z.B. eine zentrifugale Abtrennung und eine Trocknung erleichtert.

Die Konzentration der Säure beträgt vorzugsweis 5-50 Vol.%.

Während der Säurezugabe kann eine Vermischung der Suspension erfolgen, um ein schnelles Einstellen des pH-Werts und eine homogene Verteilung in der Suspension zu erreichen.

Alternativ kann anstelle einer Säure unter Einstellen des pH-Werts auch ein proteinkoagulatbildendes Salt, z.B. Calziumsulfat, Calciumchlorid, Magnesiumchlorid und/oder Calziumglyconat verwendet werden.

Die Konzentration des zugeführten Salzes bezogen auf die Gesamtmasse der Sojamilch beträgt vorzugsweise zumindest 0,15 Massen%, besonders bevorzugt zwischen 0,2 bis 2,5 Massen%.

Im Anschluss an die Säure oder Koagulatzugabe kann in einem dreizehnten Schritt eine Fällung 30 der Proteine zunächst durch Schwerkraft erfolgen. Hierfür wird die Suspension in einen Absetztank überführt. Aufgrund der pH-Wertänderung können sich die Proteine aus der Suspension absetzen. Dieser Vorgang kann vorzugsweise 5-20 min dauern. Ein optimales Absetzen der nunmehr partikulären Proteine kann bei Temperaturen von mehr als 40°C, vorzugsweise zwischen 50-80°C, besonders bevorzugt zwischen 60-75°C, erfolgen.

Nach dem Fällen erfolgt in einem vierzehnten Schritt eine Trennung 16 in eine Proteinfraktion 17 und eine Flüssigfraktion 18. Dies kann in einer Zentrifuge, insbesondere in einem Separator, oder in einer Filtrationsanlage erfolgen. Optional kann auch an dieser Stelle eine Abtrennung einer Ölphase 19 bzw. Ölfraktion als weiteres Wertprodukt erfolgen, allerdings können die Ölbestandteile auch in der Flüssigphase verbleiben. Dieses Abtrennen der Ölfraktion 19 kann alternativ oder zusätzlich zum Abtrennen der Ölfraktion 28 im zehnten Schritt 27 erfolgen.

Mit der Trennung 16 kann vorzugsweise innerhalb von 60 min, vorzugsweise innerhalb von 30 min, nach dem Einstellen des pH-Werts begonnen werden. Das Fällen von Protein zur Ausbildung einer Proteilphase und die Trennung, z.B. durch Zentrifugieren, können auch zum selben Zeitpunkt erfolgen, z.B. durch Einführen von Säure oder von koagulierendem Salz in eine Zentrifuge bzw. einen Separator oder Dekanter.

Die abgetrennte Proteinphase 17 weist vorzugsweise einen Lecithingehalt von weniger als 0,5 % Gew. auf.

Danach kann in einem fünfzehnten Schritt eine Trocknung 20 der Proteinphase 17 erfolgen. Dabei kann eine Sprühtrocknung oder besonders produktschonend eine Mahltrocknung angewandt werden.

Die so gewonnene ölfreie Proteinphase kann als Proteinpulver 32 insbesondere in der Nahrungs- und Futtermittelindustrie eingesetzt werden.

Ölfrei wird im Rahmen der vorliegenden Erfindung eine Proteinphase bezeichnet, mit weniger als 3 Gew.%, insbesondere weniger als 1 Gew.%, Öl in der getrockneten Proteinphase aufweist.

Die Zugabe des wässrigen Alkohols erfolgt vorteilhaft mit einem Alkohol-Anteil in Vol.% von 50 bis 80%.

Die bereitgestellte Sojamilch kann vorteilhaft, vorzugsweise durch ein Einengen unter Reduzierung des Wassergehalts der Sojamilch, auf einen Trockensubstanzgehalt von mehr als 12 %, vorzugsweise mehr als 15 % eingestellt werden.

Der Gehalt des wäßrigen Alkohols in der Suspension nach Schritt ii kann mehr als 20 Vol. %, vorzugsweise zwischen 25-45 Vol.%, betragen.

Nach der Zugabe des wässrigen Alkohols in Schritt ii., insbesondere unmittelbar nach der Zugabe des wäßrigen Alkohols, kann als eine Abfolge aus zumindest einem Malaxieren, vorzugsweise mit einer Rührgeschwindigkeit von weniger als 100 U/min, erfolgen.

Zumindest ein Intensiv-Rühren kann, vorzugsweise mit einer Rührgeschwindigkeit von mehr als 500 U/min, vorzugsweise von zumindest 1000 U/min, besonders bevorzugt zwischen 1000-12000 U/min, erfolgen.

Das Malaxieren kann bei 40-70°C erfolgen, und wobei besonders bevorzugt das Malaxieren in einem Zeitintervall zwischen 5-30 min erfolgen kann.

Vor Schritt iv, und nach der Abfolge aus zumindest einem Malaxieren und zumindest einem Intensiv-Rühren, kann das Abtrennen einer Ölphase aus der Suspension erfolgen.

Das Abtrennen der Ölphase vor Schritt iv. kann durch ein zentrifugales Abtrennen, insbesondere durch einen Dekanter oder einen Separator, erfolgen, wobei das zentrifugale Abtrennen besonders bevorzugt bei einer Temperatur von mehr als 20°C, vorzugsweise zwischen 35-80°C, besonders bevorzugt zwischen 35-45°C, erfolgt.

Vor Schritt iv, und vorzugsweise nach dem zentrifugalen Abtrennen der Ölphase kann eine Entalkoholisierung, vorzugsweise durch Verdampfen des Alkohols, erfolgen.

Das Fällen kann bei einer Temperatur von mehr als 20°C, vorzugsweise zwischen 50-80°C, besonders bevorzugt zwischen 60-75°C, erfolgen, wobei das Fällen besonders bevorzugt in einem Zeitraum zwischen 5-20 min erfolgt.

Das Abtrennen in Schritt v. kann ein zentrifugales Abtrennen sein und vorzugsweise durch einen Dekanter durchgeführt wird, wobei das zentrifugale Abtrennen (16), besonders bevorzugt in einer Zentrifuge oder einem Dekanter mit einem Abtrennbereich, dem sogenannten cut-off value, zwischen 1,0 bis 100 µm, erfolgen.

Das polare Lösungsmittel vor Schritt i. zur Bildung des fließfähigen Breis bei der Bereitstellung von Sojamilch kann Wasser sein, wobei das Volumen an Wasser so gewählt wird, dass sich ein Verdünnungsfaktor von mehr als 2,0, vorzugsweise zumindest 3,0, einstellt.

Eine besonders bevorzugte Variante des Verfahrens weist die folgende Schrittabfolge auf:
i. Bereitstellen der Sojamilch, vorzugsweise von mehr als pH=7,2
ii. Einengen der Sojamilch unter Erhöhung des Trockensubstanzgehalts;
iii. Zugabe des wäßrigen kurzkettigen Alkohols mit 1-5 Kohlenstoffatomen unter Ausbildung einer wässrig-alkoholischen Suspension, insbesondere unter Zugabe von Ethanol und/oder Isopropanol, wobei der Alkoholgehalt der alkoholisch-wässrigen Suspension nach Schritt iii zumindest 15 Vol. %, vorzugsweise zumindest 20 Vol.%, beträgt,
iii.i Abfolge aus zumindest einem Malaxieren mit einer Rührgeschwindigkeit von weniger als 100 U/min und/oder zumindest einem Intensiv-Rühren mit einer Rührgeschwindigkeit von zumindest 500 U/min;
iii.ii Abtrennen, insbesondere erstes zentrifugales Abtrennen, von Öl aus der Suspension unter Ausbildung einer im Wesentlichen ölfreien Suspension mit einem Ölgehalt von weniger als 5 Gew.%, vorzugsweise weniger als 3 Gew.% in der Trockenmasse
iii.iii Optionales Verringern des Alkoholgehalts, vorzugsweise durch Destillation, besonders bevorzugt unter Rückgewinnung von Alkohol;
iv. Einstellen der Suspension auf einen pH-Wert von weniger als pH=7, vorzugsweise auf einen pH-Wert zwischen pH=3,5 bis pH=6; unter Ausbildung zumindest einer Proteinphase und zumindest einer Flüssigphase;
iv.i Optionales Fällen der Proteinphase als Feststoffphase;
v. Abtrennen, insbesondere zweites zentrifugales Abtrennen, einer Proteinphase mit einem Restölgehalt von unter 5 Gew.% aus der Suspension, vorzugsweise zwischen 1-3 Gew.%, bezogen auf die Trockensubstanz der Proteinphase, sowie
vi. Trocknen der Proteinphase zu einem Proteinpulver.

Weiterhin beschrieben ist ein Verfahren zur Gewinnung von Proteinen aus einem nativen Stoffgemenge aus Soja, wobei das native Stoffgemenge zunächst zerkleinert und durch pH-Werteinstellung und Zugabe eines polaren Lösungsmittels zu einem fließfähigen alkalischen Brei verarbeitet wird, der zusätzlich zu Polarlipiden auch Proteine, Lecithin und Feststoffe enthält, gekennzeichnet durch die folgenden Schritte:
A Bearbeitung des Breis unter Ausbildung zweier voneinander getrennter Fraktionen in Form von Okara und von Sojamilch und
B Verarbeitung der Sojamilch zu einer Proteinphase nach dem zuvor beschriebenen erfindungsgemäßen Verfahren.

Das Zerkleinern des nativen Stoffgemenges kann vorteilhaft durch eine Nassvermahlung erfolgen.

Weiterhin beschrieben ist ein Sojaproteinpulver, insbesondere hergestellt nach dem erfindungsgemäßen Verfahren, mit einem Ölgehalt von max. 5% Öl in der Trockenmasse und einem Proteingehalt von mind. 70% Protein in der Trockenmasse, wobei das Sojaproteinpulver absolut hexanfrei und etherfrei ist.

### Bezugszeichen

- 1: Soja
- 2: Lagersilo
- 3: Reinigungsanlage
- 4: Aufweichen
- 5: Wasser
- 6: Zerkleinern
- 7: pH-Wert Erhöhung
- 8: NaOH- oder NaHCO₃-Lösung
- 9: Alkalischer Brei
- 10: Trennen
- 11: Okara
- 12: Sojamilch
- 13: pH-Wert Senkung
- 14: Säure
- 15: Organisches Lösungsmittel
- 16: Trennen
- 17: Proteinphase
- 18: Flüssigphasen
- 19: Ölphase
- 20: Trocknen
- 21: Einengen der Sojamilch
- 22: Verdrängungsextraktion
- 23: Abfolge
- 24: Malaxieren
- 25: Intensiv-Rühren
- 26: Malaxieren
- 27: Abtrennen
- 28: Ölphase
- 29: Entalkoholisieren
- 30: Fällen
- 31: ölfreie Suspension
- 32: Proteinpulver

## Patentansprüche

1. Verfahren zur Gewinnung von Proteinen (17) aus Sojamilch (12), umfassend
i. Bereitstellen von Sojamilch (12)
ii. Zugabe eines wäßrigen Alkohols (15) mit einer Konzentration von weniger als 80 Vol.% zur Sojamilch unter Ausbildung einer organisch-wässrigen Suspension derart, dass durch die Zugabe wäßrigen Alkohols eine Verschiebung des Löslichkeitsgleichgewichtes stattfindet und eine Verdrängungsextraktion (22) erfolgt, wobei das Volumen des wäßrigen Alkohols, so gewählt ist, dass der Gehalt des organischen Lösungsmittels der organisch-wässrigen Suspension nach Schritt ii. zumindest 15 Vol. % beträgt und dass der TS-Gehalt der Suspension mindestens 5 % beträgt;
iii. Abtrennen einer Ölphase (28) aus der Suspension;
iv. Koagulieren der Proteine unter Ausbildung zumindest einer Proteinphase (17) durch Einstellen (13) der Suspension auf einen pH-Wert von weniger als pH=7 oder durch Zugabe eines proteinkoagulatbildenden Salzes
und
v. Abtrennen (16) der Proteinphase (17) mit einem Restölgehalt von unter 5 Gew.%, bezogen auf den Trockensubstanzgehalt der Proteinphase (17), aus der Suspension.

2. Verfahren nach Anspruch 1, **dadurch gekennzeichnet, dass** das Bereitstellen von Sojamilch (12) in Schritt i. durch Gewinnung von Sojamilch (12) aus einem nativen Stoffgemenge aus Soja (1) erfolgt, wobei das native Stoffgemenge (1) zunächst zerkleinert (6) und vorzugsweise durch pH-Werteinstellung (7) auf einen basischen pH-Wert von größer als pH=7,2, besonders bevorzugt mehr als pH=8,0. und Zugabe (4) eines polaren Lösungsmittels (5), insbesondere von Wasser, zu einem fließfähigen alkalischen Brei (9) verarbeitet wird, der zusätzlich zu Lipiden auch Proteine (17), Lecithin und Feststoffe enthält, wobei die Bearbeitung des Breis (9) unter Ausbildung zweier voneinander getrennter Fraktionen in Form von Okara (11) und von Sojamilch (12) erfolgt.

3. Verfahren nach Anspruch 1 oder 2, **dadurch gekennzeichnet, dass** der wäßrige Alkohol ein aliphatischer Alkohol, insbesondere mit einer Kettenlänge von weniger als sechs-Kohlenstoff-Atomen, und/oder Isopropanol ist, wobei besonders bevorzugt die Zugabe (22) des wässrigen Alkohols mit einem Alkohol-Anteil in Vol.% von 50 bis 80% erfolgt.

4. Verfahren nach einem der vorhergehenden Ansprüche, **dadurch gekennzeichnet, dass** die bereitgestellte Sojamilch, vorzugsweise durch ein Einengen (21) unter Reduzierung des Wassergehalts der Sojamilch, auf einen Trockensubstanzgehalt von mehr als 12 %, vorzugsweise mehr als 15 % eingestellt ist.

5. Verfahren nach einem der vorhergehenden Ansprüche, **dadurch gekennzeichnet, dass** der Gehalt des wäßrigen Alkohols (15) in der Suspension nach Schritt ii mehr als 20 Vol. %, vorzugsweise zwischen 25-45 Vol.%, beträgt.

6. Verfahren nach einem der vorhergehenden Ansprüche, **dadurch gekennzeichnet, dass** nach der Zugabe (22) des wäßrigen Alkohols (15) in Schritt ii., insbesondere unmittelbar nach der Zugabe des wäßrigen Alkohols, eine Abfolge (23) aus zumindest einem Malaxieren (26), vorzugsweise mit einer Rührgeschwindigkeit von weniger als 100 U/min, erfolgt.

7. Verfahren nach einem der vorhergehenden Ansprüche, **dadurch gekennzeichnet, dass** zumindest ein Intensiv-Rühren (25), vorzugsweise mit einer Rührgeschwindigkeit von mehr als 500 U/min, vorzugsweise von zumindest 1000 U/min, besonders bevorzugt zwischen 1000-12000 U/min, erfolgt.

8. Verfahren nach einem der vorhergehenden Ansprüche, **dadurch gekennzeichnet, dass** das Malaxieren (24 und/oder 26) bei 40-70°C erfolgt, und wobei besonders bevorzugt
das Malaxieren (24 und/oder 26) in einem Zeitintervall zwischen 5-30 min erfolgt.

9. Verfahren nach einem der vorhergehenden Schritte, **dadurch gekennzeichnet, dass** vor Schritt iv, und nach der Abfolge aus zumindest einem Malaxieren (24, 26) und zumindest einem Intensiv-Rühren (25), das Abtrennen einer Ölphase (28) aus der Suspension erfolgt.

10. Verfahren nach einem der vorhergehenden Ansprüche, **dadurch gekennzeichnet, dass** das Abtrennen (27) der Ölphase (28) vor Schritt iv. durch ein zentrifugales Abtrennen (27), insbesondere durch einen Dekanter oder einen Separator, erfolgt, wobei das zentrifugale Abtrennen (27) besonders bevorzugt bei einer Temperatur von mehr als 20°C, vorzugsweise zwischen 35-80°C, besonders bevorzugt zwischen 35-45°C, erfolgt.

11. Verfahren nach einem der vorhergehenden Ansprüche, **dadurch gekennzeichnet, dass** vor Schritt iv, und vorzugsweise nach dem zentrifugalen Abtrennen (27) der Ölphase (28) eine Entalkoholisierung (29), vorzugsweise durch Verdampfen des Alkohols (15), erfolgt.

12. Verfahren nach einem der vorhergehenden Ansprüche, **dadurch gekennzeichnet, dass** das Fällen (30) bei mehr bei einer Temperatur von mehr als 20°C, vorzugsweise zwischen 50-80°C, besonders bevorzugt zwischen 60-75°C, erfolgt, wobei das Fällen (30) besonders bevorzugt in einem Zeitraum zwischen 5-20 min erfolgt.

13. Verfahren nach einem der vorhergehenden Ansprüche, **dadurch gekennzeichnet, dass** das Abtrennen (16) in Schritt v. ein zentrifugales Abtrennen (16) ist und vorzugsweise durch einen Dekanter durchgeführt wird, wobei das zentrifugale Abtrennen (16), besonders bevorzugt in einer Zentrifuge oder einem Dekanter mit einem Abtrennbereich, dem sogenannten cut-off value, zwischen 1,0 bis 100 µm, erfolgt.

14. Verfahren nach einem der vorhergehenden Ansprüche, **dadurch gekennzeichnet, dass** das polare Lösungsmittel (5) vor Schritt i. zur Bildung des fließfähigen Breis (9) bei der Bereitstellung von Sojamilch (12) Wasser (5) ist, wobei das Volumen an Wasser (5) so gewählt wird, dass sich ein Verdünnungsfaktor von mehr als 2,0, vorzugsweise zumindest 3,0, einstellt.

15. Verfahren nach einem der vorhergehenden Ansprüche, mit der folgenden Schrittabfolge:
i. Bereitstellen der Sojamilch (12), vorzugsweise von mehr als pH=7,2
ii. Einengen (21) der Sojamilch (12) unter Erhöhung des Trockensubstanzgehalts;
iii. Zugabe (22) des wäßrigen kurzkettigen Alkohols mit 1-5 Kohlenstoffatomen unter Ausbildung einer wässrig-alkoholischen Suspension, insbesondere unter Zugabe von Ethanol und/oder Isopropanol, wobei der Alkoholgehalt der alkoholisch-wässrigen Suspension nach Schritt iii zumindest 15 Vol. %, vorzugsweise zumindest 20 Vol.%, beträgt,
iii.i Abfolge (23) aus zumindest einem Malaxieren (24,26) mit einer Rührgeschwindigkeit von weniger als 100 U/min und/oder zumindest einem Intensiv-Rühren (25) mit einer Rührgeschwindigkeit von zumindest 500 U/min;
iii.ii Abtrennen (27), insbesondere erstes zentrifugales Abtrennen, von Öl aus der Suspension unter Ausbildung einer im Wesentlichen ölfreien Suspension mit einem Ölgehalt von weniger als 5 Gew.%, vorzugsweise weniger als 3 Gew.% in der Trockenmasse
iii.iii Optionales Verringern des Alkoholgehalts (29), vorzugsweise durch Destillation, besonders bevorzugt unter Rückgewinnung von Alkohol;
iv. Einstellen (13) der Suspension auf einen pH-Wert von weniger als pH=7, vorzugsweise auf einen pH-Wert zwischen pH=3,5 bis pH=6; unter Ausbildung zumindest einer Proteinphase und zumindest einer Flüssigphase (18);
iv.i Optionales Fällen (30) der Proteinphase als Feststoffphase;
v. Abtrennen (16), insbesondere zweites zentrifugales Abtrennen, einer Proteinphase (17) mit einem Restölgehalt von unter 5 Gew.% aus der Suspension, vorzugsweise zwischen 1-3 Gew.%, bezogen auf die Trockensubstanz der Proteinphase (17), sowie
vi. Trocknen (20) der Proteinphase (17) zu einem Proteinpulver.

## Claims

1. Method for obtaining proteins (17) from soy milk (12), comprising
i. provision of soy milk (12);
ii. addition of an aqueous alcohol (15) with a concentration of less than 80% by volume to the soy milk with formation of an organic-aqueous suspension in such a way that a shift in the solubility equilibrium takes place due to the addition of aqueous alcohol and a displacement extraction (22) takes place, wherein the volume of the aqueous alcohol is selected in such a way that the content of the organic solvent of the organic-aqueous suspension after step ii. is at least 15% by volume and that the dry substance content of the suspension is at least 5%;
iii. separation of an oil phase (28) from the suspension;
iv. coagulation of the proteins to form at least one protein phase (17) by adjusting (13) the suspension to a pH value of less than pH=7 or by adding a protein-coagulate-forming salt;
and
v. separation (16) of the protein phase (17) with a residual oil content of less than 5 wt.%, based on the dry substance content of the protein phase (17), from the suspension.

2. Method according to claim 1, **characterized in that** the provision of soy milk (12) in step i. is carried out by obtaining soy milk (12) from a native mixture of substances from soy (1), wherein the native mixture of substances (1) is first comminuted (6) and is processed preferably by pH adjustment (7) to a basic pH value of greater than pH=7.2, particularly preferably greater than pH=8.0, and addition (4) of a polar solvent (5), in particular water, to form a flowable alkaline slurry (9) which, in addition to lipids, also contains proteins (17), lecithin and solids, wherein the processing of the slurry (9) is carried out with the formation of two separate fractions in the form of okara (11) and of soy milk (12).

3. Method according to claim 1 or 2, **characterized in that** the aqueous alcohol is an aliphatic alcohol, in particular with a chain length of less than six carbon atoms, and/or isopropanolwherein the addition (22) of the aqueous alcohol is carried out with an alcohol content in percent by volume of 50 to 80% is particularly preferred.

4. Method according to one of the preceding claims, **characterized in that** the prepared soy milk is adjusted to a dry substance content of more than 12%, preferably more than 15%, preferably by concentrating (21) while reducing the water content of the soy milk.

5. Method according to one of the preceding claims, **characterized in that** the content of the aqueous alcohol (15) in the suspension after step ii is more than 20% by volume, preferably between 25-45% by volume.

6. Method according to one of the preceding claims, **characterized in that** after the addition (22) of the aqueous alcohol (15) in step ii., in particular immediately after the addition of the aqueous alcohol, a sequence (23) of at least one malaxation (26) is carried out, preferably at a stirring speed of less than 100 rpm.

7. Method according to one of the preceding claims, **characterized in that** at least one intensive stirring (25) is carried out, preferably with a stirring speed of more than 500 rpm, preferably of at least 1000 rpm, particularly preferably between 1000-12000 rpm.

8. Method according to one of the preceding claims, **characterized in that** the malaxation (24 and/or 26) is carried out at 40-70°C, and wherein the malaxation (24 and/or 26) is performed in a time interval between 5-30 min is particularly preferred.

9. Method according to one of the preceding steps, **characterized in that** before step iv, and after the sequence of at least one malaxation (24, 26) and at least one intensive stirring (25), the separation of an oil phase (28) from the suspension is carried out.

10. Method according to one of the preceding claims, **characterized in that** the separation (27) of the oil phase (28) prior to step iv. is carried out by a centrifugal separation (27), in particular by a decanter or a separator, wherein the centrifugal separation (27) is carried out particularly preferred at a temperature higher than 20°C, preferably between 35-80°C, more preferably between 35-45°C.

11. Method according to one of the preceding claims, **characterized in that** before step iv, and preferably after centrifugal separation (27) of the oil phase (28), dealcoholization (29) is carried out, preferably by evaporation of the alcohol (15).

12. Method according to one of the preceding claims, **characterized in that** the precipitation (30) is carried out at more at a temperature higher than 20°C, preferably between 50-80°C, more preferably between 60-75°C, wherein the precipitation (30) is carried out particularly preferred in a period of time between 5-20 min.

13. Method according to one of the preceding claims, **characterized in that** the separation (16) in step v. is a centrifugal separation (16) and is preferably carried out by a decanter, wherein the centrifugal separation (16) is carried out particularly preferred in a centrifuge or decanter with a separation range, the so-called cut-off value, between 1.0 to 100 µm.

14. Method according to one of the preceding claims, **characterized in that** the polar solvent (5) prior to step i. for forming the flowable slurry (9) in the provision of soy milk (12) is water (5), wherein the volume of water (5) is selected such that a dilution factor of more than 2.0, preferably at least 3.0, is obtained.

15. A method according to one of the preceding claims, comprising the following sequence of steps:
i. provision of the soy milk (12), preferably of more than pH=7.2;
ii. concentration (21) of the soy milk (12) while increasing the dry matter content;
iii. addition (22) of the aqueous short-chain alcohol having 1-5 carbon atoms to form an aqueous-alcoholic suspension, in particular with the addition of ethanol and/or isopropanol, wherein the alcohol content of the alcoholic-aqueous suspension after step iii is at least 15% by volume, preferably at least 20% by volume,
iii.i sequence (23) of at least one malaxation (24,26) with a stirring speed of less than 100 rpm and/or at least one intensive stirring (25) with a stirring speed of at least 500 rpm;
iii.ii separating (27), in particular first centrifugal separating, of oil from the suspension to form a substantially oil-free suspension having an oil content of less than 5 wt.%, preferably less than 3 wt.% in dry matter;
iii.iii optional reduction of the alcohol content (29), preferably by distillation, particularly preferably with recovery of alcohol;
iv. adjusting (13) the suspension to a pH value of less than pH=7, preferably to a pH value between pH=3.5 to pH=6; by forming at least one protein phase and at least one liquid phase (18);
iv.i optional precipitation (30) of the protein phase as a solid phase;
v. separating (16), in particular second centrifugal separating, of a protein phase (17) with a residual oil content of less than 5 wt.% from the suspension, preferably between 1-3 wt.%, based on the dry substance of the protein phase (17), as well as
vi. drying (20) the protein phase (17) to a protein powder.

## Revendications

1. Procédé pour l'extraction de protéines (17) de lait de soja (12), comprenant
i. la préparation de lait de soja (12) ;
ii. l'addition d'un alcool aqueux (15) à une concentration inférieure à 80 % en volume au lait de soja en formant une suspension organique aqueuse, de sorte que l'ajout d'alcool aqueuse provoque un décalage de l'équilibre de solubilité et qu'une extraction par refoulement (22) se produit, le volume de l'alcool aqueux étant choisi de telle sorte que la teneur en solvant organique de la suspension organique aqueuse soit d'au moins 15 % en volume et le taux de matière sèche dans la suspension d'au moins 5 % après l'étape ii ;
iii. séparation d'une phase huileuse (28) de la suspension ;
iv. coagulation des protéines avec formation d'au moins une phase protéique (17) par ajustement (13) de la suspension à un pH inférieur à pH 7 ou par addition d'un sel coagulant les protéines
et
v. séparation (16) de la suspension de la phase protéique (17) avec une teneur en huile résiduelle inférieure à 5 % en poids par rapport à la teneur en matière sèche de la phase protéique (17).

2. Procédé selon la revendication 1, **caractérisé en ce que** le lait de soja (12) est préparé dans l'étape i. par extraction de lait de soja (12) d'un mélange natif de substances issues de soja (1), le mélange natif de substances (1) étant d'abord broyé (6) et de préférence ajusté à un pH basique supérieur à pH 7,2, en particulier supérieur à pH 8,0, par ajustement du pH (7) et additionné (4) d'un solvant polaire (5), en particulier d'eau, pour donner une bouillie alcaline fluide (9) contenant, outre les lipides, des protéines (17), de la lécithine et des solides, le traitement de la bouillie (9) produisant deux fractions séparées sous forme d'okara (11) et de lait de soja (12).

3. Procédé selon la revendication 1 ou 2, **caractérisé en ce que** l'alcool aqueux est un alcool aliphatique, ayant en particulier une longueur de chaîne de moins de six atomes de carbone, et/ou de l'isopropanol, l'alcool aqueux étant de préférence ajouté (22) à raison de 50 à 80 % en volume d'alcool.

4. Procédé selon l'une des revendications précédentes, **caractérisé en ce que** le lait de soja préparé est ajusté, de préférence par réduction (21) avec diminution de sa teneur en eau, à une teneur en matière sèche supérieure à 12 %, de préférence supérieure à 15 %.

5. Procédé selon l'une des revendications précédentes, **caractérisé en ce que** la teneur en alcool aqueux (15) de la suspension de l'étape ii est supérieure à 20 % du volume, de préférence comprise entre 25 et 45 % du volume.

6. Procédé selon l'une des revendications précédentes, **caractérisé en ce que** l'ajout (22) de l'alcool aqueux (15) dans l'étape ii. est suivi, en particulier immédiatement après l'ajout de l'alcool aqueux, d'une séquence (23) d'au moins un malaxage (26), de préférence à une vitesse d'agitation inférieure à 100 tours/minute.

7. Procédé selon l'une des revendications précédentes, **caractérisé en ce qu'**au moins un agitation intensive (25) est effectuée, de préférence à une vitesse d'agitation supérieure à 500 tours/minute, de préférence d'au moins 1000 tours/minute, en particulier comprise entre 1000 et 12 000 tours/minute.

8. Procédé selon l'une des revendications précédentes, **caractérisé en ce que** le malaxage (24 et/ou 26) est effectué entre 40 et 70 °C et dans lequel le malaxage (24 et/ou 26) est effectué, de préférence, dans un intervalle de temps de 5 à 30 minutes.

9. Procédé selon l'une des revendications précédentes, **caractérisé en ce que** la séparation d'une phase huileuse (28) de la suspension est effectuée avant l'étape iv et après la séquence d'au moins un malaxage (24, 26) et au moins un agitation intensive (25).

10. Procédé selon l'une des revendications précédentes, **caractérisé en ce que** la séparation (27) de la phase huileuse (28) est effectuée avant l'étape iv. par séparation centrifuge (27), en particulier par un décanteur ou un séparateur, la séparation centrifuge (27) étant de préférence réalisée à une température supérieure à 20 °C, en particulier entre 35 et 80 °C et notamment entre 35 et 45 °C.

11. Procédé selon l'une des revendications précédentes, **caractérisé en ce qu'**une extraction de l'alcool (29), de préférence par évaporation de l'alcool (15), est effectuée avant l'étape iv et de préférence après la séparation centrifuge (27) de la phase huileuse (28).

12. Procédé selon l'une des revendications précédentes, **caractérisé en ce que** la précipitation (30) est effectuée à une température supérieure à 20 °C, de préférence entre 50 et 80 °C, en particulier entre 60 et 75 °C, la précipitation (30) étant effectuée de préférence dans un intervalle de temps de 5 à 20 minutes.

13. Procédé selon l'une des revendications précédentes, **caractérisé en ce que** la séparation (16) de l'étape v. est une séparation centrifuge (16) réalisée de préférence par un décanteur, la séparation centrifuge (16) étant en particulier réalisée dans une centrifugeuse ou un décanteur avec une plage de séparation (*cut-off value*) comprise entre 1,0 et 100 µm.

14. Procédé selon l'une des revendications précédentes, **caractérisé en ce que** le solvant polaire (5) utilisé avant l'étape i. pour la formation de la bouillie fluide (9) lors de la préparation du lait de soja (12) est de l'eau (5), le volume d'eau (5) étant choisi de façon à obtenir un facteur de dilution supérieur à 2,0, de préférence d'au moins 3,0.

15. Procédé selon l'une des revendications précédentes, comprenant la séquence d'étapes suivante :
i. préparation du lait de soja (12), de préférence à plus de pH 7,2 ;
ii. réduction (21) du lait de soja (12) avec augmentation de la teneur en matière sèche ;
iii. ajout (22) de l'alcool à chaîne courte aqueux possédant entre 1 et 5 atomes de carbone avec formation d'une suspension alcoolique aqueuse, en particulier par ajout d'éthanol et/ou d'isopropanol, la teneur en alcool de la suspension alcoolique aqueuse après l'étape iii étant d'au moins 15 % en volume, de préférence d'au moins 20 % en volume ;
iii.i séquence (23) d'au moins un malaxage (24, 26) à une vitesse d'agitation inférieure à 100 tours/minute et/ou d'au moins une agitation intensive (25) à une vitesse d'agitation d'au moins 500 tours/minute ;
iii.ii séparation (27) d'huile de la suspension, en particulier première séparation centrifuge, pour former une suspension sensiblement déshuilée contenant une teneur en huile inférieure à 5 %, de préférence inférieure à 3 % en poids de la masse sèche ;
iii.iii réduction facultative de la teneur en alcool (29), de préférence par distillation, en particulier avec récupération de l'alcool ;
iv. ajustement (13) du pH de la suspension à moins de pH 7, de préférence à un pH compris entre pH 3,5 et pH 6, avec formation d'au moins une phase protéique et au moins une phase liquide (18) ;
iv.i précipitation (30) facultative de la phase protéique formant la phase solide ;
v. séparation (16) de la suspension, en particulier deuxième séparation centrifuge, d'une phase protéique (17) ayant une teneur en huile résiduelle inférieure à 5 % en poids, de préférence entre 1 et 3 % en poids par rapport à la matière sèche de la phase protéique (17), et
vi. séchage (20) de la phase protéique (17) pour obtenir une poudre de protéines.
